# EUROPEAN PATENT SPECIFICATION

(11) **EP 2 315 552 B1**
(45) Date of publication and mention of the grant of the patent: **30.11.2011**
(21) Application number: 09809413.9
(22) Date of filing: 24.08.2009
(51) Int. Cl.: A61B 17/72, A61B 17/74

(54) **OSTEOSYNTHESIS DEVICE MAKING IT POSSIBLE TO REPLACE AN ARTICULAR BONE HEAD ON THE CORRESPONDING BONE**
OSTEOSYNTHESEVORRICHTUNG ZUR ERSETZUNG EINES GELENKKNOCHENKOPFS AUF DEM ENTSPRECHENDEN KNOCHEN
DISPOSITIF D OSTÉOSYNTHÈSE PERMETTANT LE REMPLACEMENT D UNE TÊTE D OS ARTICULAIRE SUR L OS CORRESPONDANT

(30) Priority: 26.08.2008 FR 0855725
(43) Date of publication of application: 04.05.2011
(73) Proprietor: FX Solutions, 01000 Bourg en Bresse (FR)
(72) Inventor: LASCAR, Tristan, F-06320 Cap D'ail (FR); MARTIN, Jean-Jacques, F-01000 Bourg En Bresse (FR)
(74) Representative: Jeannet, Olivier
(86) International application number: PCT/IB2009/053713
(87) International publication number: WO 2010/023614

(56) References cited:
- WO-A-2005/070314
- WO-A-2007/046691
- FR-A- 2 669 528
- US-A- 5 300 074

## Description

The present invention concerns an osteosynthesis device making it possible to replace an articular bone head on the corresponding bone, in particular to treat a fracture of the humerus.

It is known from document FR 2 669 528, in the name of Levon Doursounian, to replace the articular head of a humerus on the rest of the bone using an osteosynthesis device. This device comprises a bone anchoring portion, in particular in the form of a medullary rod, and an anchoring plate, or support portion, designed to support the native articular head, these anchoring and support portions being designed to be assembled to one another by an assembly system of low slope cones. In order to maintain the humeral head thereon, said support portion comprises peripheral points capable of being inserted into the cancellous bone of the humeral head.

This known device also includes a prosthetic articular head which can be assembled to the anchoring portion instead and in place of the support portions and native humeral head when reuse of this native head is impossible or undesirable.

This existing material has the determining advantage of allowing a practitioner the possibility of choosing whether or not to keep the humeral head. Preserving this humeral head is desirable when possible, given that it avoids having to implement a prosthetic cavity and consequently simplifies the operation considerably.

However, this material has the drawback of requiring the use of a prosthetic head in a certain number of cases in which the condition of the native head could, however, allow this native head to be reused. This is in particular the case when the native head has a fragility or reduced quantity of cancellous bone making its perfect maintenance on the support portion uncertain.

The present invention aims to resolve this drawback.

The osteosynthesis device which it concerns comprises, in a known manner, a bone anchoring portion, in particular in the form of a medullary rod, and a support portion designed to support the native articular head, these anchoring and support portions being designed to be assembled to one another by an assembly system; said support portion comprises a central plate and at least one peripheral plate with an axis parallel to the central plate.

According to the invention,
- the central plate has at least one peripheral flange forming an inclined anterior face, which allows the engagement of the central plate in the cancellous bone of the bone head by insertion of the plate in this bone, and a posterior face substantially perpendicular to the axis of the plate, designed to bear against the cancellous bone in order to ensure that the bone head is kept on said support portion, and
- said peripheral plate has at least one peripheral flange which forms an inclined anterior face, allowing the engagement of the peripheral plate in the cancellous bone of the bone head by insertion of the plate in this bone, and a posterior face substantially perpendicular to the axis of the peripheral plate, designed to bear against the cancellous bone in order to ensure retention of the bone head on said support portion.

It will be understood that "anterior" and "posterior" are defined in relation to the direction of insertion of the central plate and the peripheral plate in the cancellous bone of the bone head.

Said central plate is thus both central and retentive, and is able to rest perfectly in the zone of the bone head comprising the greatest thickness of cancellous bone; simultaneously, the peripheral plate rests in the cancellous bone of the bone head in a peripheral location thereof, and allows lateral stabilization of the bone head in relation to said support portion while increasing the bearing surface of this support portion in the cancellous bone.

Perfect retention of the native bone head on said support portion is thus ensured, without involving complex operations to assemble this head to the support portion. Once this assembly is done, the structure is assembled to said bone anchoring portion, also secured to the bone.

The central portion may comprise only one peripheral flange; preferably, however, it comprises several such flanges, stepped over its length.

The central plate preferably has a transverse section such that it occupies between approximately 1/10^{th} and 1/6^{th} of the surface of a base wall comprised by the support portion.

This central plate thus has a significant dimension in transverse section, allowing it to bear against the cancellous bone via a significant surface, which favors good retention of the bone head on the support portion.

According to another preferred embodiment of the invention, said plate has a widened free end, forming a peripheral flange which defines a peripheral posterior face.

This posterior face has an extended surface, also favorable to good retention of the bone head on the support portion.

Said widened free end advantageously has a pointed form favoring the insertion of the plate into the cancellous bone, in particular a conical form.

The central plate can define a low slope conical cavity, for wedgingly receiving a complementary conical tip comprised by said anchoring portion.

The support portion advantageously comprises several peripheral plates, in particular regularly distributed on a circle concentric to the central plate.

The support portion comprises a base wall including said central plate and the peripheral plate(s), and this base wall has openings arranged through it, allowing the passage of bone cells.

The invention will be well understood, and other characteristics and advantages thereof will appear, in reference to the appended diagrammatic drawing, non-limitingly illustrating one preferred embodiment of the osteosynthesis device it concerns.
Figure 1 is a perspective view of a support portion comprised by this device;
figure 2 is a side view;
figure 3 is a front view, and
figures 4 and 5 are views of the device before and after performing the osteosynthesis of a humerus fractured at the articulation head.

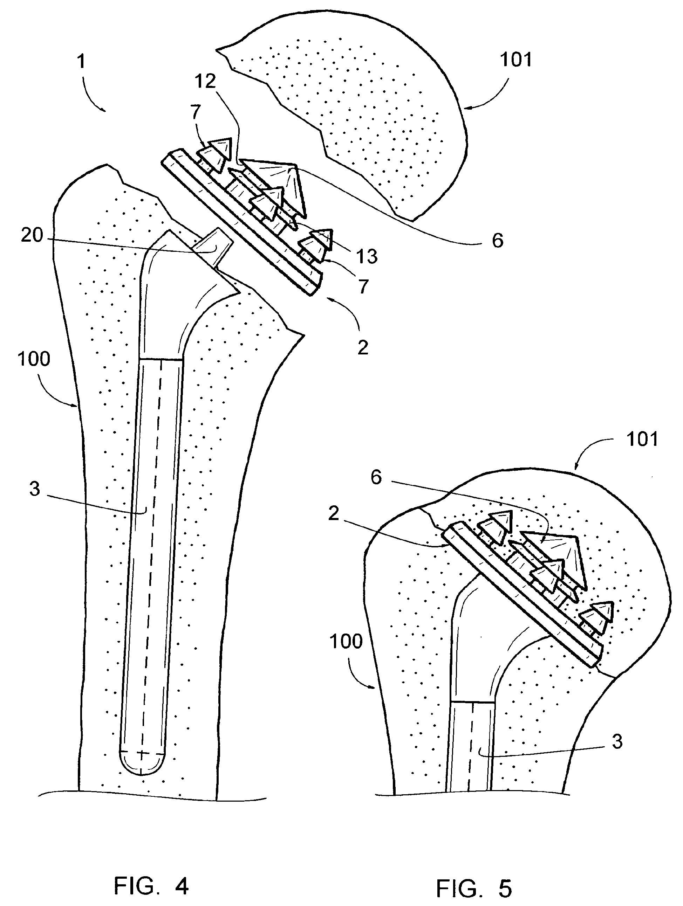

Figures 4 and 5 illustrate an osteosynthesis device 1 making it possible to replace the fractured head 101 of a humerus on the rest of the humerus 100.

This device 1 comprises an anchoring plate 2, or "support portion", and an anchoring portion 3 in the form of a medullary rod.

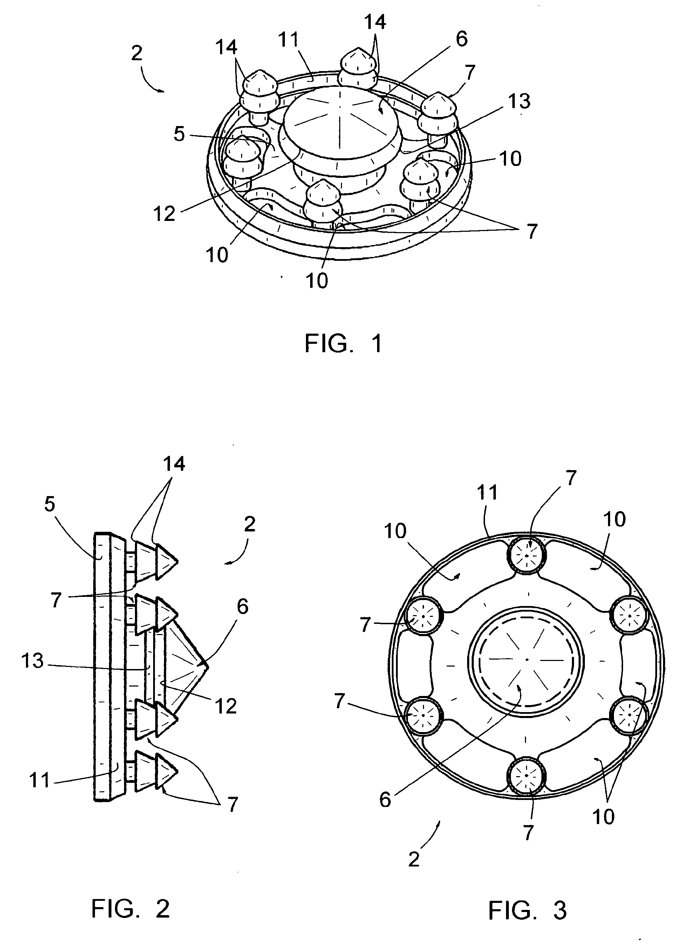

In reference to figures 1 to 3, it appears that the support portion 2 comprises a base plate 5, a central plate 6 and six peripheral plates 7, this central plate 6 and these peripheral plates 7 protruding from one of the main faces of the plate 5. The assembly is formed in a stiff biocompatible material, in particular titanium.

The base plate 5 is circular and has a diameter adapted to insertion into the cancellous bone present at the fractured face of the head 101, as shown by figures 4 and 5.

It comprises, between each peripheral plate 7, lumens 10 going all the way through it. These lumens 10 enable substantial connection of the two main faces opposite the plate 5 in order to allow, after replacement of the head 101 on the humerus 100, the passage of blood and growing bone cells through the plate 5. In the illustrated embodiment, these openings 10 are located close to the peripheral edge of the plate 5 and are shaped like ring portions, extending over the majority of the zones separating the consecutive peripheral plates 7.

The base plate 5 also comprises a peripheral rim 11.

The central plate 6 is circular and has a transverse section clearly greater than that of the peripheral plates 7. Its transverse section is such that it occupies approximately 1/7^{th} of the surface of the base plate 5.

It has a widened free end, conical in shape, forming a peripheral flange 12 and, at approximately two-thirds of the height of the plate 6, a peripheral flange 13.

These flanges 12 and 13 each form an inclined anterior face, which makes it possible to engage the central plate 6 in the cancellous bone of the bone head by insertion of this plate 6 into this bone, and a posterior face substantially perpendicular to the axis of the plate, designed to bear against the cancellous bone in order to ensure retention of the bone head 101 on said support portion 2.

The plate 6 inwardly defines a low slope conical cavity, which leads to the face of the wall 5 opposite that comprising this plate 6, this conical cavity being designed to wedgingly receive a complementary conical tip 20 comprised by the medullary rod 3.

The peripheral plates 7 have axes parallel to that of the central plate 6 and protrude from the same face of the plate 5 as that from which the plate 6 protrudes.

These plates 7 have a height slightly smaller than that of the plate 6 (cf. figure 2) and are regularly distributed on a circle concentric to this plate 6 (cf. figure 3).

Each plate 7 forms, at its free end, two stepped conical flanges 14 which define anterior and posterior peripheral faces as previously mentioned.

The medullary rod 3 is of the traditional type, configured to be inserted into the medullary cavity of the humerus 100. It is made in a stiff biocompatible material, in particular titanium, and can be sealed using a synthetic cement or comprise an osteo-inducer coating, in particular a porous calcium hydroxyapatite coating.

In practice, the medullary rod 3 is placed on the humerus 100 and the support portion 2 is engaged in the head 101, then the support portion 2 - head 101 assembly is assembled to the rod 3 by insertion and wedging of the tip 20 in the cavity defined by the plate 6.

The peripheral flanges 12, 13 and 14 constitute support surfaces configured so as not to present an obstacle to the engagement of the plates 6 and 7 in the cancellous bone of the head 101, by insertion of these plates in this bone; after this engagement, these support surfaces bear against the cancellous bone in order to ensure retention of the head 101 on the support portion 2.

The pointed shapes of the plate 6 and protrusions 14 favor the insertion of the plates 6, 7 in the cancellous bone.

The plate 6, which is both central and retentive, is able to rest perfectly in the zone of the head 101 comprising the greatest thickness of cancellous bone; its significant dimension in transverse section and its two peripheral flanges enable it to bear against the cancellous bone via significant areas, favorable to good retention of the head 101 on the support portion 2.

The plates 7 increase the support surface of the support portion 2 in the cancellous bone and allow lateral stabilization of the head 101 in relation to this support portion 2.

It appears from the preceding that the invention provides an osteosynthesis material making it possible to replace an articular bone head on the corresponding bone, in particular of a fractured humeral head on the humerus, presenting the determining advantages of ensuring perfect retention of the native bone head on said support portion without involving complex operations in order to assemble this head to the support portion.

This perfect retention makes it possible to preserve the bone head as much as possible and avoids resorting to the use of a prosthetic head in a certain number of cases, in particular when the native bone head is fragile or has a reduced quantity of cancellous bone.

The invention was described above in reference to an embodiment provided as an example. It goes without saying that it is not limited to this embodiment and that it extends to all other embodiments and variations covered by the appended claims.

## Claims

1. Osteosynthesis device (1) making it possible to replace an articular bone head (101) on the corresponding bone, comprising a bone anchoring portion (3), in particular in the form of a medullary rod, and a support portion (2) designed to support the native articular head (101), these anchoring and support portions (3, 2) being designed to be assembled to one another by an assembly system; said support portion (2) comprises a central plate (6) and at least one peripheral plate (7) with an axis parallel to the central plate (6)
**characterized in that**:
- the central plate (6) has at least one peripheral flange (12, 13) forming an inclined anterior face, which allows the engagement of the central plate (6) in the cancellous bone of the bone head (101) by insertion of the plate (6) in this bone, and a posterior face substantially perpendicular to the axis of the plate, designed to bear against the cancellous bone in order to ensure that the bone head (101) is kept on said support portion, and
- said peripheral plate (7) has at least one peripheral flange (14) which forms an inclined anterior face, allowing the engagement of the peripheral plate (7) in the cancellous bone of the bone head (101) by insertion of the plate (7) in this bone, and a posterior face substantially perpendicular to the axis of the peripheral plate (7), designed to bear against the cancellous bone in order to ensure retention of the bone head (101) on said support portion.

2. Device (1) according to claim 1, **characterized in that** the central portion (6) comprises several peripheral flanges (12, 13), stepped over its length.

3. Device (1) according to claim 1 or claim 2, **characterized in that** the central portion (6) has a transverse section such that it occupies between approximately 1/10^{th} and 1/6^{th} of the surface of a base wall (5) comprised by the support portion (2).

4. Device (1) according to any of claims 1 to 3, **characterized in that** the central portion (6) has a widened free end, forming a peripheral flange (12) which defines a peripheral posterior face.

5. Device (1) according to claim 4, **characterized in that** said widened free end has a pointed form favoring the insertion of the central plate (6) into the cancellous bone, in particular a conical form.

6. Device (1) according to any of claims 1 to 5, **characterized in that** the central plate (6) defines a low slope conical cavity, for wedgingly receiving a complementary conical tip (20) comprised by said anchoring portion (3).

7. Device (1) according to any of claims 1 to 6, **characterized in that** the support portion (2) comprises several peripheral plates (7), in particular regularly distributed on a circle concentric to the central plate (6).

8. Device (1) according to any of claims 1 to 7, **characterized in that** the support portion (2) comprises a base wall (5) including said central plate (6) and the peripheral plate(s) (7), and **in that** this base wall (5) has openings (10) arranged through it.

## Patentansprüche

1. Osteosynthesevorrichtung (1), die es ermöglicht, einen Gelenkknochenkopf (101) auf dem entsprechenden Knochen zu ersetzen, umfassend ein Knochen-Verankerungsteil (3), insbesondere in Form eines Knochenmark-Stabs, und ein Trägerteil (2), der ausgestaltet ist, den natürlichen Gelenkkopf (101) zu tragen, wobei diese Verankerungs- und Trägerteile (3, 2) ausgestaltet sind, mit einem Montagesystem aneinander montiert zu werden; das Trägerteil (2) umfasst eine Zentralplatte (6) und mindestens eine periphere Platte (7) mit einer zur Zentralplatte (6) parallelen Achse
**dadurch gekennzeichnet, dass**:
- die Zentralplatte (6) mindestens einen peripheren Flansch (12, 13) aufweist, der eine geneigte vordere Fläche bildet, die das Eingreifen der Zentralplatte (6) in die Knochenschwammsubstanz des Knochenkopfes (101) durch Einsetzen der Platte (6) in diesen Knochen ermöglicht, und eine hintere Fläche, die im Wesentlichen senkrecht zu der Achse der Platte ist, die ausgestaltet ist, an der Knochenschwammsubstanz anzuliegen, um zu gewährleisten, dass der Knochenkopf (101) auf dem Trägerteil gehalten wird, und
- die periphere Platte (7) mindestens einen peripheren Flansch (14) aufweist, der eine geneigte vordere Fläche bildet, die das Eingreifen der peripheren Platte (7) in die Knochenschwammsubstanz des Knochenkopfes (101) durch Einsetzen der Platte (7) in diesen Knochen ermöglicht, und eine hintere Fläche, die im Wesentlichen senkrecht zu der Achse der peripheren Platte (7) ist, die ausgestaltet ist, an der Knochenschwammsubstanz anzuliegen, um das Halten des Knochenkopfes (101) auf dem Trägerteil zu gewährleisten.

2. Vorrichtung (1) nach Anspruch 1, **dadurch gekennzeichnet, dass** das Zentralteil (6) abgestuft über seine Länge mehrere periphere Flansche (12, 13) umfasst.

3. Vorrichtung (1) nach Anspruch 1 oder Anspruch 2, **dadurch gekennzeichnet, dass** das Zentralteil (6) einen solchen Querschnitt hat, dass er zwischen 1/10 und 1/6 der Fläche einer Bodenwand (5) einnimmt, die das Trägerteil (2) umfasst.

4. Vorrichtung (1) nach einem der Ansprüche 1 bis 3, **dadurch gekennzeichnet, dass** das Zentralteil (6) ein verbreitertes freies Ende aufweist, das einen peripheren Flansch (12) bildet, der eine periphere hintere Fläche bestimmt.

5. Vorrichtung (1) nach Anspruch 4, **dadurch gekennzeichnet, dass** das verbreiterte freie Ende eine spitze Form, insbesondere eine konische Form, aufweist, die das Einsetzen der Zentralplatte (6) in die Knochenschwammsubstanz begünstigt.

6. Vorrichtung (1) nach einem der Ansprüche 1 bis 5, **dadurch gekennzeichnet, dass** die Zentralplatte (6) einen flach abfallenden konischen Hohlraum bestimmt, um eine komplementäre konische Spitze (20), die aus dem Verankerungsteil (3) besteht, durch Verkeilung aufzunehmen.

7. Vorrichtung (1) nach einem der Ansprüche 1 bis 6, **dadurch gekennzeichnet, dass** das Trägerteil (2) mehrere periphere Platten (7) umfasst, die insbesondere regelmäßig auf einem Kreis konzentrisch zur Zentralplatte (6) verteilt sind.

8. Vorrichtung (1) nach einem der Ansprüche 1 bis 7, **dadurch gekennzeichnet, dass** das Trägerteil (2) eine Bodenwand (5), umfassend die Zentralplatte (6) und die periphere(n) Platte(n) (7), umfasst, und dass die Bodenwand (5) Öffnungen (10) hat, die durch diese hindurch angeordnet sind.

## Revendications

1. Matériel d'ostéosynthèse (1) permettant la remise en place d'une tête osseuse articulaire (101) sur l'os correspondant, comprenant une partie d'ancrage (3) à l'os, notamment sous forme d'une tige médullaire, et une partie de support (2) destinée à supporter la tête articulaire native (101), ces parties d'ancrage et de support (3, 2) étant destinées à être assemblées l'une à l'autre par un système d'assemblage ; ladite partie de support (2) comprend un plot central (6) et au moins un plot périphérique (7) d'axe parallèle au plot central (6) ;
**caractérisé en ce que** :
- le plot central (6) présente au moins une collerette périphérique (12, 13) formant une face antérieure inclinée, qui permet l'engagement du plot central (6) dans l'os spongieux de la tête osseuse (101) par insertion de ce plot (6) dans cet os, et une face postérieure sensiblement perpendiculaire à l'axe du plot, destinée à prendre appui contre l'os spongieux afin d'assurer la rétention de la tête osseuse (101) sur ladite partie de support, et
- ledit plot périphérique (7) présente au moins une collerette périphérique (14) formant une face antérieure inclinée, qui permet l'engagement du plot périphérique (7) dans l'os spongieux de la tête osseuse (101) par insertion de ce plot (7) dans cet os, et une face postérieure sensiblement perpendiculaire à l'axe du plot périphérique (7), destinée à prendre appui contre l'os spongieux afin d'assurer la rétention de la tête osseuse (101) sur ladite partie de support.

2. Matériel (1) selon la revendication 1, **caractérisé en ce que** le plot central (6) comprend plusieurs collerettes périphériques (12, 13), étagées sur sa longueur.

3. Matériel (1) selon la revendication 1 ou la revendication 2, **caractérisé en ce que** le plot central (6) a une section transversale telle qu'il occupe entre environ 1/10^{e} et 1/6^{e} de la surface d'une paroi de base (5) que comprend la partie de support (2).

4. Matériel (1) selon l'une des revendications 1 à 3, **caractérisé en ce que** le plot (6) présente une extrémité libre élargie, formant une collerette périphérique (12) qui délimite une face postérieure périphérique.

5. Matériel (1) selon la revendication 4, **caractérisé en ce que** ladite extrémité libre élargie présente une forme pointue favorisant l'insertion du plot central (6) dans l'os spongieux, notamment une forme conique.

6. Matériel (1) selon l'une des revendications 1 à 5, **caractérisé en ce que** le plot central (6) délimite une cavité conique à faible pente, de réception avec coincement d'un embout conique complémentaire (20) que comprend ladite partie d'ancrage (3).

7. Matériel (1) selon l'une des revendications 1 à 6, **caractérisé en ce que** la partie de support (2) comprend plusieurs plots périphériques (7), notamment régulièrement répartis sur un cercle concentrique au plot central (6).

8. Matériel (1) selon l'une des revendications 1 à 7, **caractérisé en ce que** la partie de support (2) comprend une paroi de base (5) comportant le plot central (6) et ou les plots périphériques (7), et **en ce que** cette paroi de base (5) présente des ouvertures (10) aménagées au travers d'elle.
